# EUROPEAN PATENT SPECIFICATION

(11) **EP 2 303 287 B1**
(45) Date of publication and mention of the grant of the patent: **11.12.2013**
(21) Application number: 09772137.7
(22) Date of filing: 30.06.2009
(51) Int. Cl.: A61K 31/731, A61P 11/06, A61P 27/14, A61P 37/08

(54) **ANTIALLERGIC MARINE BIOPOLYMERS**
ANTIALLERGISCHE MARINE BIOPOLYMERE
BIOPOLYMÈRES ANTIALLERGIQUES MARINS

(30) Priority: 01.07.2008 US 129507 P
(43) Date of publication of application: 06.04.2011
(73) Proprietor: Marinomed Biotechnologie GmbH, 1210 Vienna (AT)
(72) Inventor: GRASSAUER, Andreas, A-1220 Vienna (AT); PRIESCHL-GRASSAUER, Eva, A-1220 Vienna (AT)
(74) Representative: Bogensberger, Burkhard
(86) International application number: PCT/EP2009/004696
(87) International publication number: WO 2010/000437

(56) References cited:
- EP-A- 1 930 015
- WO-A-2008/015007
- WO-A-2008/067982
- WO-A-2009/027057
- JP-A- 2003 081 841
- NICKLIN S ET AL: "Iota-carrageenan induced reaginic antibody production in the rat - I. Characterisation and kinetics of the response" INTERNATIONAL JOURNAL OF IMMUNOPHARMACOLOGY, ELMSFORD,NY, US, vol. 7, no. 5, 1 January 1985 (1985-01-01), pages 677-685, XP023812676 ISSN: 0192-0561 [retrieved on 1985-01-01]

## Description

### FIELD OF INVENTION

The present invention is in the field of physiology and immunology and relates to the use of iota-carrageenan for the prophylactic or therapeutic treatment of allergies.

### STATE OF THE ART

Structurally, the carrageenans are a complex group of polysaccharides made up of repeating galactose-related monomer units. Currently, three main types of carrageenans are distinguished, namely lambda, kappa, and iota carrageenan. The lambda form does not gel strongly at room temperature and therefore allows administration by injection, for example in order to induce an inflammatory response. Inflammation induced by carrageenan is well researched and highly reproducible. Cardinal signs of inflammation, i.e. edema, hyperalgesia, and erythema develop immediately following subcutaneous injection of lambda carrageenan and typically result from the action of proinflammatory agents including bradykinin, histamine, tachykinins, reactive oxygen and nitrogen species that emerge in situ at the site of insult or by infiltrating cells.

The inflammatory response is usually quantified in a rat paw inflammation model by determining the increase in rat paw size (edema size) which reaches a maximum at about 5 h post injection of an inflammatory agent such as lambda carrageenan or another antigen and is modulated by inhibitors of specific molecules within the inflammatory cascade. This model for determining the intensity of an inflammation has been relied upon in more than 1000 scientific publications in the evaluation of potential anti-inflammatory agents.

Allergies have an increasing incidence in the western hemisphere with about 20% of the population being affected now. An allergy can refer to several kinds of unwanted immune reactions including type I, type III, or type IV hypersensitivities. While in type I and type III allergies granulocytes, a subset of leukocytes, are involved in the pathogenesis, in type IV allergies T-cells are the major cause of these diseases. Mast cells and basophiles are the cellular basis for type I allergies, i.e. IgE-mediated allergies via the Fc[epsilon]RI.

Allergies of all types can result in symptoms ranging from hardly more than a runny nose to severe chronic diseases and even life-threatening anaphylactic shock or septic shock.

Type I allergies are commonly treated by corticosteroids (e.g. cortisol), antihistamines, epinephrine, theophylline or mast cell stabilizers. These compounds block the action of allergic mediators, preventing activation of cells and degranulation processes. These drugs help alleviate the symptoms of acute allergy but play a little role in the therapy of chronic allergic disorders. All of the aforementioned drugs entail quite substantial side-effects, especially after long-term use.

Allergic rhinitis is an immunological disorder and an inflammatory response of nasal mucosal membranes. It represents a state of hypersensitivity and occurs when the body overreacts in response to a mucosally ingested substance such as pollen or dust.

The airway epithelium is normally protected from dehydration and from inhaled infectious or toxic agents by the presence of a sound mucus layer. Mucus also plays a very important role as a mechanical barrier or filter system in preventing inhaled particles from reaching the sensitive alveoli of the lungs.

Airway mucus is a complex mixture of proteins, enzymes, lipids and a sol phase composed of water and electrolytes. Some 95% of mucus is water and this water is bound in a viscoelastic gel containing mucins, which are large, i.e. high molecular weight glycoproteins. As a result of the low surface tension of the mucus, the particles and infectious agents sucked in through the nose are immediately adsorbed and get trapped by the mucus.

Patients who suffer from allergic rhinitis and asthma, as well as other conditions associated with inhaled allergens often have a reduced amount of mucus or mucus with abnormal properties. When the mucus layer is damaged, interrupted, or poorly developed and therefore its adsorption capacity is significantly impaired allergenic particles my pass through that leaky barrier and reach the mast cells of the nasal cavity where they start triggering an allergic reaction.

Nasal mucus is constantly being removed from the nasal tract by gravity as well as by mucociliary clearance, which removal is an important element of the defense of the ciliated nasal epithelium against inhaled allergens and infectious agents. Patients suffering from allergic rhinitis frequently have abnormally slow or prolonged mucociliary clearance which may contribute to their increased sensitivity towards allergens.

WO 2008/067982 A discloses an anti-rhinoviral pharmaceutical composition comprising carrageenan, preferably iota-carrageenan, together with another pharmaceutically active compound, e.g. cortisone or an antihistamine for prophylactic or therapeutic treatment of various conditions including allergy, wherein in such a composition the carrageenan exerts anti-rhinoviral adjuvant function.

JP 2003 081841 A discloses the use of lambda- and/or kappa-carrageenan for the treatment of allergic diseases, wherein the anti-allergic composition is administered orally in the form of a food or drinking article.

### DESCRIPTION OF THE INVENTION

The scope of the invention is defined by the claims.

It has now surprisingly been found that iota and kappa carrageenan but not lambda carrageenan can be used to inhibit the inflammatory response of mast cells and thus provide a therapy for the prevention and treatment of type I allergies.

Also, it has been found that mucociliary clearance can be normalized, i.e. adjusted to a normal clearance rate, by administering compositions comprising such carrageenans which can help controlling the humidity in the nasal tract.

In a typical embodiment of the present invention iota-carrageenan is present as a therapeutically active agent in an antiallergic pharmaceutical composition and exerts upon mucosal administration a local effect such as, for example, in the respiratory tract, the gastrointestinal tract, or the eyes. However, where the iota-carrageenan reaches the bloodstream via the mucosal way an additional systemic effect may occur.

According to the present invention the active antiallergic ingredient iota carrageenan may be formulated into any pharmaceutical composition suitable for mucosal administration to a recipient, comprising liquid solutions of variable viscosities, foams, gels, creams, drops, lozenges, tablets, capsules, chewing gum, and powders.

The anti-allergic pharmaceutical compositions referred to herein comprise iota carrageenan but are substantially free from lambda carrageenan. In this context "substantially free" means that the concentration of lambda carrageenan is as low as caused by usual impurities of commercially available iota carrageenan. Typically, the amount of lambda carrageenan impurities does not exceed 5 %, and usually not even 2% by weight relative to the total of iota- carrageenan in the compositions of the present invention. Where lambda carrageenan is expressis verbis disclaimed from the pharmaceutical compositions of the present inventions it is to be understood that the compositions are free from "substantial" amounts of lambda carrageenan, as herein before defined.

The pharmaceutical compositions of the present invention referred to herein may be available with or without prescription by a medical doctor and may encompass compositions that require marketing approval from the Medicines and Healthcare Products Regulatory Agency.

The present invention is further illustrated by the following figures and examples.

### BRIEF DESCRIPTION OF THE FIGURES

- Fig.1: represents a diagrammatic view of the inhibition of TNF-alpha production from IgE-antigen stimulated mast cells. The Y-axis depicts the concentration of TNF-alpha in pg/ml.
1 = non-stimulated, 2= IgE/antigen stimulated, 3= lambda-carrageenan pretreated, 4 = kappa-carrageenan pretreated, 5 = iota-carrageenan pretreated.
- Fig.2.: represents a diagrammatic view on the dose-dependent inhibition of TNF-alpha production from IgE/antigen stimulated mast cells. The Y-axis depicts the concentration of TNF-alpha in pg/ml.

### EXAMPLES

Example 1: Inhibition of TNF-alpha production from IgE/antigen stimulated mast cells.

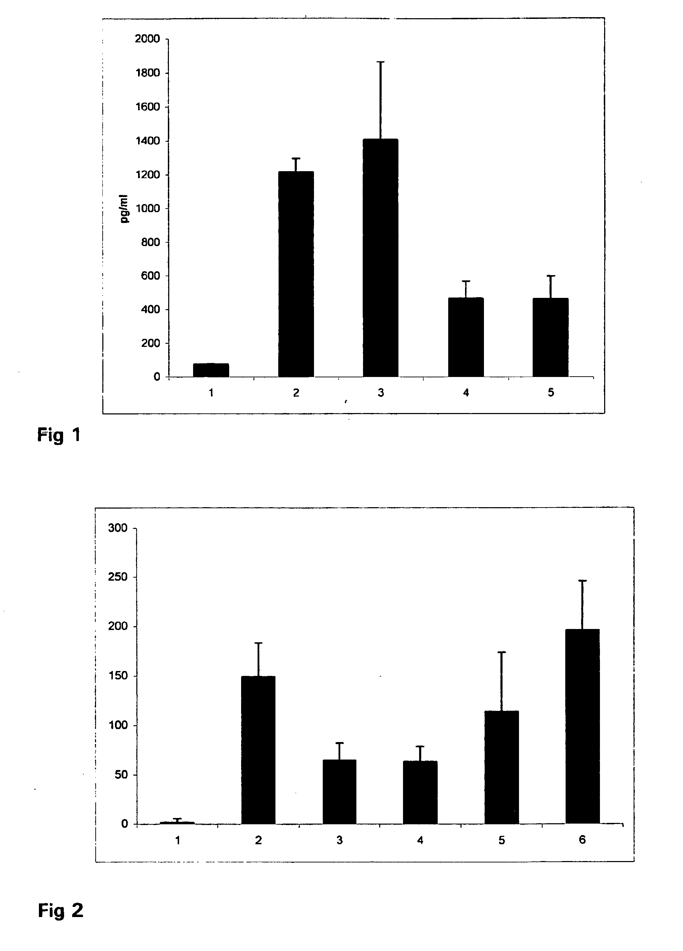

TNF-alpha is a mediator that is central in an inflammatory process as observed during infections, and autoimmune diseases. It is released by white blood cells, mast cells, endothelium and several other tissues in the course of damage. Cell based assays using mouse mast cells stimulated with IgE/antigen complex demonstrated that both iota and kappa Carrageenan but not lambda Carrageenan inhibit TNF-alpha release (Fig 1.).

CFTL12 mast cells were incubated with iota or kappa carrageenan at a concentration of 200µg/ml. 60 minutes later the cells were stimulated with an IgE/antigen complex. Cells were incubated at 37°C for 6 hours and TNF-alpha in the supernatant was determined by a commercial mouse TNF-alpha ELISA (Bender-Med-Systems). Error bars indicate the standard deviation between 4 independent wells. 1 = non-stimulated; 2 = IgE / antigen stimulation; 3 = lambda carrageenan pretreatment+subsequent IgE/antigen stimulation; 4= kappa carrageenan pretreatment + subsequent IgE/antigen stimulation, 5 = iota-carrageenan pretreatment+subsequent IgE/antigen stimulation. The Y-axis mirrors the concentration of TNF-alpha in pg/ml.

### Example 2: Dose-dependent inhibition of TNF-alpha production from IgE/antigen stimulated mast cells

Mast cells were incubated with iota-carrageenan at varying concentrations. 60 minutes later the cells were stimulated with IgE/antigen complex. Cells were incubated at 37°C for 6 hours and TNF-alpha in the supernatant was determined by a commercial mouse TNF-alpha ELISA (Bender-Med-Systems). The results are shown in Fig. 2. Error bars indicate the standard deviation between 4 independent wells. 1 = non-stimulated; 2 = IgE/ antigen stimulated, 3 = iota-carrageenan 200 µg/ml, 4 = iota-carrageenan 66 µg/ml, 5 = iota-carrageenan 6.6 µg/ml, 6 = iota-carrageenan 0.6 µg/ml. The Y-axis represents the concentration of TNF-alpha in pg/ml.

### Example 3: Application of a carrageenan nasal spray for the improvement of allergic symptoms.

A 29 year old patient with a proven history of type I allergy with strong symptoms of allergic rhinitis and an allergy against several kinds of plant pollen was subjected to the following treatment regime: a nasal spray containing 0.12% iota-carrageenan was administered daily in the evening and the dosage was increased during the pollen season. The patient reported a strong reduction in sneezing frequency when the nasal spray was used and undisturbed sleeping was possible again. In addition, the patient reported a reduction of the inflammation of the nasal mucosa. The patient further reported that additional medication especially intranasal decongestants, antihistamines and corticosteroids, was no longer required, very much in contrast to the preceding pollen seasons.

### Example 4: Anti-allergic prophylactic effect

A 26 year old volunteer was asked to apply a nasal spray containing 0.12% iota-carrageenan with the intention to provide prophylaxis against common cold infections. In compliance with the administration protocol the volunteer sprayed at least twice daily for several months. He had a history of severe common cold infections and a history of allergic reaction against several types of plant pollen. Surprisingly, the volunteer reported that in spite of a rather severe pollen season he, contrary to expectation, did not suffer from any symptoms of allergic rhinitis (hay fever). However, the volunteer reported symptoms of allergic reaction in other parts of the body where the nasal spray was not applied. These symptoms included itching of eyes and redness of the skin. It may thus be inferred from these observations that repeated topic, i.e. mucosal administration of a iota-carrageenan composition according to the present invention, for example by way of a nasal spray, may convey prophylactic and/or therapeutic protection to individuals at risk of acquiring allergic rhinitis (hay fever). Since plant pollen is mainly released during the spring season afflicted individuals may thus be able to relieve the symptoms of hay fever or possibly to entirely prevent an outbreak of hay fever by timely starting to prophylactically administer the nasal spray of the present invention.

### Example 5: Mucosal delivery of non-carrageenan physiologically active compounds

A solution comprising 0.12% wt of iota-carrageenan in phosphate buffered hypophysiologic, i.e. 0.5%, saline was supplemented with 0.09 % wt of escin. 300 µl (containing 270 µg escin) of the solution were transferred into one nostril of New Zealand White Rabbits four times daily for five days. After the last application, plasma was collected from the animals at the time points 30 min, 1 h, 2 h, 4 h, 6 h, and 12 h. The plasma concentration of escin peaked at 6 h and reached maximum concentrations of up to about 90 ng/ml. This concentration is significantly higher compared to those obtained after oral application in humans. Plasma levels of escin in human patients treated repeatedly with 50 mg escin twice a day were lower than 20 ng/ml (10 ng/ml average level, 16-18 ng/ml maximum concentration). These results indicate that carrageenan may exert an adjuvant function upon combined mucosal administration with a desired non-carrageenan active substance such as escin. Carrageenan may thus be used in the manufacture of pharmaceutical compositions suitable for mucosal application, for improving the bioavailability of physiologically active compounds that are only poorly bioavailable upon oral administration.

The carrageenan-based pharmaceutical compositions of the present invention may comprise a suitable carrier, as well as additional active or non-active ingredients. Where the iota carrageenan is the sole active ingredient it is contained in the composition at a concentration effective for providing an antiallergic effect upon topical, e.g. mucosal administration.

Typically, the concentration of iota-carrageenan in liquid, gel-like, solid or powder compositions may range from 0.05 - 5 % by weight, concentrations between 0.1 - 2% wt being preferred.

In one embodiment, the pharmaceutical composition may comprise one or more non-carrageenan active ingredients and may be adjusted to provide sustained release of such ingredients upon intranasal administration. For example, such non-carrageenan active ingredients may be released over a period of 30 minutes, 1 hour, 2 hours, 4, 6, 8, 10 or 12 hours from intranasal administration.

Such active ingredients may preferably be selected from the group of herbal or homeopathic agents rather than from pharmaceutically active drugs. Herbal or homeopathic remedies usually do not exhibit any or at most minimal toxicity at the concentrations required to produce a therapeutic effect.

In this context, the terms "homeopathic" and "herbal" shall refer to products derived from natural plant or mineral sources.

For example, escin, the major active principle from the horse chestnut tree (*Aesculus hippocastanum*), was shown to have clinically significant activity in the treatment of chronic venous insufficiency (CVI), hemorrhoids and postoperative edema. In a study wherein 50mg tablets were administered to volunteers peak levels of 16-18 ng escin per 1 ml were detected in the blood plasma. It was now surprisingly found that via the intranasal administration of escin in combination with iota-carrageen (0.09% wt escin, 0.12% wt iota-carrageenan, in 0.5% hypophysiological NaCl solution) similar or even higher plasma levels can be reached in a rabbit animal model (data not shown). Thus the mucosal, e.g. intranasal administration of desired homeopathic or other low or non-toxic compounds having only a very poor bioavailability in the human body upon oral intake may be substantially improved by administering such compounds in combination with iota-carrageenan as some sort of adjuvant.

Non-carrageenan active ingredients that may be present in the compositions of the present invention may be selected from the group consisting of homeopathic or herbal remedies having one or more of the following properties: antibacterial and/or antifungal, antiviral, antibiotic, anti-inflammatory, anti-insomnia, cognitive enhancing, or properties that affect cardiovascular function, such as cardiotonic properties, antidysrythmic or antianginal properties, vasoconstriction or vasodilatation properties or anti-hypertensive properties.

Specific examples may be selected from the group consisting of aspirin; St John's Wort; valerian extract which may include sesquiterpenes, valeric acid, iridoids, valepotriates, alkaloids, furanofuran lignans, amino acids, [gamma]-aminobutyric acid, tyrosine, arginine, glutamine or any combination thereof; ginkgo biloba extract which may include flavonoids, ginkgolides and bilobalides or any combination thereof; vitamins A, E or C; garlic, lime, one or more pro-biotics, ginger, ellagic acid, echinacea, Swedish flower pollen, black walnut hulls, lemongrass, wormwood, grapefruit seed extract, broccoli, digestive enzymes, hyaluronic acid, astralgus, rosehips, gentian, hypericum, horse chestnut, ginseng, green tea, phosphatidyl serine, phosphatidyl choline, citrus, pycnogenol, caffeine, quercitin, co-enzyme Q, yarrow, tea tree, noni juice (Morinda citrifolia), lipase, fructo-oligosaccharide, inulin, black cumin (Nigella sativa) or allicin.

Compositions for use according to the present invention may comprise one or more of these non-carrageenan ingredients, provided that the selected ingredients are compatible with one another under conditions of storage and use.

Compositions for use according to the present invention may further comprise kali bichromium; a thickening agent such as a gum or starch; a disintegrant, such as sodium starch glycolate or cross-linked povidone; a release agent such as magnesium stearate; an emulsifying agent; a surfactant; pharmaceutically acceptable excipients; anti-caking agents; granulating agents; preservatives; such colorants as may be desired or any combination thereof.

Where the composition is in the form of a powder, it is preferred that the powder composition does not include components which, although frequently used in intranasal compositions, can cause irritation or affect ciliary movement such as, for example, solvents like propylene glycol, absorption enhancers like cyclodextrins or glycosides, or mucoadhesives such as chitosan.

Compositions for use according to the present invention may further comprise a flavoring additive such as menthol, mint, spearmint, peppermint, eucalyptus, lavender, citrus, lemon, lime, or any combination thereof.

The inclusion of flavors in an orally or nasally administrable pharmaceutical composition usually confers a pleasant sensory feedback on the recipient, which frequently improves the patient's recollection of administration times and thus can improve his compliance with the administration regime. More specifically, compositions including mint, menthol and the like seem to be more effective in treating allergic rhinitis and asthma than compositions of the invention which do not include a flavor.

Preparations for use according to the present invention which include mint seem to also have an advantageous effect on the airways facilitating easier and smoother breathing, which is particularly beneficial with patients suffering from asthma. Also, patients of a nervous disposition tend to breathe in an irregular pattern. The administration of carrageenan preparations including flavors such as mint can provide a feel-good sensation which may contribute to restoring normal breathing patterns.

In another embodiment the present invention relates to a pharmaceutical composition for combined use of iota-carrageenan, flavoring agent and non-carrageenan active ingredient. lota-carrageenan, flavoring agent and non-carrageenan active ingredient may be included together in a single preparation, or alternatively, be provided in separate preparations each for sequential administration. Where administration is sequential, iota-carrageenan and/or flavoring agent may be administered separately or together before or after the non-carrageenan active ingredient, or both before and thereafter. Similarly, the non-carrageenan active ingredient may be administered before or after or both before and after the combined or separate administration of iota-carrageenan and flavoring agent.

## Claims

1. Iota-carrageenan in an anti-allergic effective amount for use as a medicament in the prophylactic or therapeutic treatment of allergic rhinitis, allergic conjunctivitis, or allergic asthma, wherein said medicament is for administration to mucosal surfaces of the respiratory tract or the eyes, with the proviso that the iota-carrageenan is substantially free of lambda-carrageenan.

2. Iota-carrageenan for use as a medicament according to claim 1, wherein said medicament is for administration to the nasal mucosa or the mucosa of the eyes.

3. Iota-carrageenan for use as a medicament according to claim 1, to restore or supplement impaired adsorption capacity of mucus.

4. Iota-carrageenan for use as a medicament according to any one of claims 1 to 3, wherein said medicament is adapted as a nasal spray.

5. Use of iota-carrageenan in an anti-allergic effective amount in the manufacture of a pharmaceutical composition for the prophylactic or therapeutic treatment of allergic rhinitis, allergic conjunctivitis, or allergic asthma, wherein said pharmaceutical composition is for administration to mucosal surfaces of the respiratory tract or the eyes, with the proviso that the iota-carrageenan is substantially free of lambda-carrageenan.

6. Use of iota-carrageenan according to claim 5, wherein said pharmaceutical composition is for administration to the nasal mucosa or the mucosa of the eyes.

7. Use of iota-carrageenan according to claim 5, to restore or supplement impaired adsorption capacity of mucus.

8. Use of iota-carrageenan according to claim 5, wherein said pharmaceutical composition comprises 0.05 to 5 % by weight, usually 0.1 - 2% wt, of iota-carrageenan.

9. Use of iota-carrageenan according to claim 5, wherein said pharmaceutical composition further comprises a non-carrageenan physiologically active ingredient.

10. Use of iota-carrageenan according to claim 9, wherein the ingredient is selected from the group of herbal or homeopathic agents derived from natural plant or mineral sources.

11. Use of iota-carrageenan according to claim 8, wherein said pharmaceutical composition further comprises a flavor, preferably selected from the group consisting of menthol, mint, spearmint, peppermint, eucalyptus, lavender, citrus, lemon, lime, or any combination thereof.

12. Use of iota-carrageenan according to claim 8, wherein said pharmaceutical composition further comprises at least one additive selected from the group consisting of kali bichromium, a thickening agent such as a gum or starch, a disintegrant, such as sodium starch glycolate or cross-linked povidone, a release agent such as magnesium stearate, an emulsifying agent, a surfactant, a pharmaceutically acceptable excipient or carrier, an anti-caking agent, a granulating agent, a preservative, a colorant.

13. Use of iota-carrageenan according to claim 5, wherein said pharmaceutical composition is formulated as a liquid solution of a preselected viscosity of 20 mPa*s or less, preferably of 1 to 10 mPa*s.

14. Use of iota-carrageenan according to any one of claims 5 to 13, wherein said pharmaceutical composition is adapted as a nasal spray.

## Patentansprüche

1. Iota-Carrageenan in einer antiallergisch wirksamen Menge zur Verwendung als Medikament bei der prophylaktischen oder therapeutischen Behandlung von allergischer Rhinitis, allergischer Konjunktivitis oder allergischem Asthma, wobei das Medikament für eine Verabreichung an Schleimhautoberflächen der Atemwege oder der Augen vorgesehen ist, mit der Maßgabe, dass das Iota-Carrageenan im Wesentlichen frei von Lambda-Carrageenan ist.

2. Iota-Carrageenan zur Verwendung als Medikament nach Anspruch 1, wobei das Medikament zur Verabreichung an die Nasenschleimhaut oder die Augenschleimhaut vorgesehen ist.

3. Iota-Carrageenan zur Verwendung als Medikament nach Anspruch 1 zur Wiederherstellung oder Ergänzung einer beeinträchtigten Adsorptionsfähigkeit von Schleim.

4. Iota-Carrageenan zur Verwendung als Medikament nach einem der Ansprüche 1 bis 3, wobei das Medikament als Nasenspray ausgelegt ist.

5. Verwendung von Iota-Carrageenan in einer antiallergisch wirksamen Menge bei der Herstellung einer pharmazeutischen Zusammensetzung für die prophylaktische oder therapeutische Behandlung von allergischer Rhinitis, allergischer Konjunktivitis oder allergischem Asthma, wobei die pharmazeutische Zusammensetzung für eine Verabreichung an Schleimhautoberflächen der Atemwege oder der Augen vorgesehen ist, mit der Maßgabe, dass das Iota-Carrageenan im Wesentlichen frei von Lambda-Carrageenan ist.

6. Verwendung von Iota-Carrageenan nach Anspruch 5, wobei die pharmazeutische Zusammensetzung für eine Verabreichung an die Nasenschleimhaut oder die Augenschleimhaut vorgesehen ist.

7. Verwendung von Iota-Carrageenan nach Anspruch 5 zur Wiederherstellung oder Ergänzung einer beeinträchtigten Adsorptionsfähigkeit von Schleim.

8. Verwendung von Iota-Carrageenan nach Anspruch 5, wobei die pharmazeutische Zusammensetzung 0,05 bis 5 Gew.-%, für gewöhnlich 0,1 bis 2 Gew.-%, Iota-Carrageenan umfasst.

9. Verwendung von Iota-Carrageenan nach Anspruch 5, wobei die pharmazeutische Zusammensetzung darüber hinaus einen physiologisch aktiven Nicht-Carrageenan-Inhaltsstoff umfasst.

10. Verwendung von Iota-Carrageenan nach Anspruch 9, wobei der Inhaltsstoff aus der Gruppe von Kräuter- und homöopathischen Mitteln ausgewählt ist, die aus natürlichen Pflanzen- oder Mineralquellen abgeleitet sind.

11. Verwendung von Iota-Carrageenan nach Anspruch 8, wobei die pharmazeutische Zusammensetzung darüber hinaus einen Geschmacksstoff umfasst, vorzugsweise ausgewählt aus der Gruppe bestehend aus Menthol, Minze, Grüner Minze, Pfefferminze, Eukalyptus, Lavendel, Zitrone, Limone, Limette oder einer Kombination davon.

12. Verwendung von Iota-Carrageenan nach Anspruch 8, wobei die pharmazeutische Zusammensetzung darüber hinaus zumindest einen Zusatzstoff umfasst, ausgewählt aus der Gruppe, bestehend aus Kalium Bichromicum, einem Verdickungsmittel wie Gummi oder Stärke, einem Sprengmittel wie Natriumstärkeglykolat oder vernetztes Povidon, einem Trennmittel wie Magnesiumstearat, einem Emulgator, einem Tensid, einem pharmazeutisch annehmbaren Exzipienten oder Träger, einer Rieselhilfe, einem Granuliermittel, einem Konservierungsmittel, einem Farbstoff.

13. Verwendung von Iota-Carrageenan nach Anspruch 5, wobei die pharmazeutische Zusammensetzung als flüssige Lösung mit einer vorab ausgewählten Viskosität von 20 mPa*s oder weniger, vorzugsweise 1 bis 10 mPa*s, formuliert ist.

14. Verwendung von Iota-Carrageenan nach einem der Ansprüche 5 bis 13, wobei die pharmazeutische Zusammensetzung als Nasenspray ausgelegt ist.

## Revendications

1. Iota-carraghénane dans une quantité efficace anti-allergique pour une utilisation comme médicament dans le traitement prophylactique ou thérapeutique de la rhinite allergique, de la conjonctivite allergique ou de l'asthme allergique, ledit médicament étant destiné à l'administration aux surfaces de muqueuses du tractus respiratoire ou des yeux, à la condition que l'iota-carraghénane soit sensiblement exempt de lambda-carraghénane.

2. Iota-carraghénane pour une utilisation comme médicament selon la revendication 1, dans lequel ledit médicament est destiné à l'administration à la muqueuse nasale ou à la muqueuse des yeux.

3. Iota-carraghénane pour une utilisation comme médicament selon la revendication 1, pour restaurer ou supplémenter la capacité d'adsorption détériorée du mucus.

4. Iota-carraghénane pour une utilisation comme médicament selon l'une quelconque des revendications 1 à 3, dans lequel ledit médicament est adapté en tant que pulvérisation nasale.

5. Utilisation d'iota-carraghénane dans une quantité efficace anti-allergique dans la fabrication d'une composition pharmaceutique pour le traitement prophylactique ou thérapeutique de la rhinite allergique, de la conjonctivite allergique ou de l'asthme allergique, dans laquelle ladite composition pharmaceutique est destinée à l'administration aux surfaces de muqueuses du tractus respiratoire ou des yeux, à la condition que l'iota-carraghénane soit substantiellement exempt de lambda-carraghénane.

6. Utilisation d'iota-carraghénane selon la revendication 5, dans laquelle ladite composition pharmaceutique est destinée à l'administration à la muqueuse nasale ou à la muqueuse des yeux.

7. Utilisation d'iota-carraghénane selon la revendication 5, pour restaurer ou supplémenter la capacité d'adsorption détériorée du mucus.

8. Utilisation d'iota-carraghénane selon la revendication 5, dans laquelle ladite composition pharmaceutique comprend 0,05 à 5 % en poids, de façon usuelle 0,1-2 % en poids, d'iota-carraghénane.

9. Utilisation d'iota-carraghénane selon la revendication 5, dans laquelle ladite composition pharmaceutique comprend en outre un principe physiologiquement actif non carraghénane.

10. Utilisation d'iota-carraghénane selon la revendication 9, dans laquelle le principe est choisi dans le groupe d'agents d'origine végétale ou homéopathiques issus de sources végétales ou minérales naturelles.

11. Utilisation d'iota-carraghénane selon la revendication 8, dans laquelle ladite composition pharmaceutique comprend en outre un arôme, choisi de préférence dans le groupe consistant en le menthol, la menthe, la menthe verte, la menthe poivrée, l'eucalyptus, la lavande, le citrus, le citron, le citron vert ou toute combinaison de ceux-ci.

12. Utilisation d'iota-carraghénane selon la revendication 8, dans laquelle ladite composition pharmaceutique comprend en outre au moins un additif choisi dans le groupe consistant en le bichromate de potassium, un agent épaississant tel qu'une gomme ou de l'amidon, un désintégrant, tel que le glycolate d'amidon sodique ou la povidone réticulée, un agent de démoulage tel que du stéarate de magnésium, un agent émulsifiant, un tensioactif, un excipient ou support pharmaceutiquement acceptable, un agent anti-agglomérant, un agent de granulation, un conservateur, un colorant.

13. Utilisation d'iota-carraghénane selon la revendication 5, dans laquelle ladite composition pharmaceutique est formulée en tant que solution liquide d'une viscosité présélectionnée de 20 mPa*s ou moins, de préférence de 1 à 10 mPa*s.

14. Utilisation d'iota-carraghénane selon l'une quelconque des revendications 5 à 13, dans laquelle ladite composition pharmaceutique est adaptée en tant que pulvérisation nasale.
